# EUROPEAN PATENT SPECIFICATION

(11) **EP 3 081 081 B1**
(45) Date of publication and mention of the grant of the patent: **23.05.2018**
(21) Application number: 14869059.7
(22) Date of filing: 10.12.2014
(51) Int. Cl.: A01K 67/027, C12N 15/09, C12N 9/64, C12N 15/85

(54) **TRANSGENIC NON-HUMAN MAMMAL EXPRESSING HUMAN MMP2**
TRANSGENES NICHT-MENSCHLICHES SÄUGETIER ZUR EXPRESSION VON MENSCHLICHEM MMP2
MAMMIFÈRE TRANSGÉNIQUE NON HUMAIN EXPRIMANT LA PROTÉINE HUMAINE MMP2

(30) Priority: 12.12.2013 JP 2013256900
(43) Date of publication of application: 19.10.2016
(73) Proprietor: Mie University, Tsu-shi, Mie 514-8507 (JP)
(72) Inventor: ESTEBAN C. Gabazza, Tsu-shi Mie 514-8507 (JP); TAGUCHI, Osamu, Tsu-shi Mie 514-8507 (JP); KOBAYASHI, Tetsu, Tsu-shi Mie 514-8507 (JP)
(74) Representative: Sutcliffe, Nicholas Robert
(86) International application number: PCT/JP2014/082691
(87) International publication number: WO 2015/087916

(56) References cited:
- WO-A2-99/31969
- JP-A- H08 511 507
- JP-A- 2007 167 060
- J. L. WRIGHT ET AL: "Animal models of chronic obstructive pulmonary disease", AMERICAN JOURNAL OF PHYSIOLOGY - LUNG CELLULAR AND MOLECULAR PHYSIOLOGY, vol. 295, no. 1, 18 April 2008 (2008-04-18), pages 1-15, XP055362550, US ISSN: 1040-0605, DOI: 10.1152/ajplung.90200.2008
- S. D. SHAPIRO: "Transgenic and gene-targeted mice as models for chronic obstructive pulmonary disease", EUROPEAN RESPIRATORY JOURNAL., vol. 29, no. 2, 27 September 2006 (2006-09-27), pages 375-378, XP055362641, DK ISSN: 0903-1936, DOI: 10.1183/09031936.00087606
- JOSEPH GEORGE ET AL: "Transgenic expression of human matrix metalloproteinase-9 augments monocrotaline-induced pulmonary arterial hypertension in mice", JOURNAL OF HYPERTENSION., vol. 29, no. 2, February 2011 (2011-02), pages 299-308, XP055362328, GB ISSN: 0263-6352, DOI: 10.1097/HJH.0b013e328340a0e4
- DAMJANOVSKI S ET AL: "Overexpression of matrix metalloproteinases leads to lethality in transgenic Xenopus laevis: implications for tissue-dependent functions of matrix metalloproteinases during late embryonic development", DEVELOPMENTAL DYNAMICS, WILEY-LISS, INC., NEW YORK, NY, US, vol. 221, no. 1, May 2001 (2001-05), pages 37-47, XP002410650, ISSN: 1058-8388, DOI: 10.1002/DVDY.1123
- BERGMAN, M. R. ET AL.: 'Cardiac matrix metalloproteinase-2 expression independently induces marked ventricular remodeling and systolic dysfunction' AMERICAN JOURNAL OF PHYSIOLOGY HEART AND CIRCULATORY PHYSIOLOGY vol. 292, pages H1847 - H1860, XP055348846
- CHENG, S. ET AL.: 'Matrix metalloproteinase 2 and basement membrane integrity: a unifying mechanism for progressive renal injury' THE FASEB JOURNAL vol. 20, 2006, pages E1248 - E1256, XP055348851
- ZHOU, H. Z. ET AL.: 'Transgenic MMP-2 expression induces latent cardiac mitochondrial dysfunction' BIOCHEMICAL AND BIOPHYSICAL RESEARCH COMMUNICATIONS vol. 358, no. 1, 22 June 2007, pages 189 - 195, XP022077628
- DAHI, S. ET AL.: 'Transgenic expression of matrix metalloproteinase-2 induces coronary artery ectasia' INTERNATIONAL JOURNAL OF EXPERIMENTAL PATHOLOGY vol. 92, 2011, pages 50 - 56, XP055348854

### SEQUENCE LISTING

<110> MIE University
<120> A transgenic animal which expresses hMMP2
<130> JP2014002MIU
<150> JP2013-256900
   <151> 2013-12-12
<160> 6
<170> PatentIn version 3.5
<210> 1
   <211> 25
   <212> DNA
   <213> Mus musculus
<400> 1
   cccttattga cctcaactac atggt 25
<210> 2
   <211> 23
   <212> DNA
   <213> Mus musculus
<400> 2
   gaggggccat ccacagtctt ctg 23
<210> 3
   <211> 20
   <212> DNA
   <213> Mus musculus
<400> 3
   caccaccgag gactatgacc 20
<210> 4
   <211> 20
   <212> DNA
   <213> Mus musculus
<400> 4
   tgttgcccag gaaagtgaag 20
<210> 5
   <211> 23
   <212> DNA
   <213> Homo sapiens
<400> 5
   tactggatct actcagccag cac 23
<210> 6
   <211> 23
   <212> DNA
   <213> Homo sapiens
<400> 6
   caggatccat tttcttcttc acc 23

## Description

### [Technical Field]

The present invention relates to transgenic (TG) mice which express a human MMP2 (metalloproteinase 2; hereinafter referred to as "hMMP2").

### [Background Art]

Matrix metalloproteinases (MMP) are a group of metalloproteinases which is a generic term for proteinases including metal ions in its active center. More than 20 types of MMPs such as MMP1, MMP2, MMP3, MMP7, MMP8, MMP9, MMP10 are known. The MMPs show actions such as degradation of an extracellular matrix including collagen, proteoglycan, elastin, etc., degradation of proteins expressed on cell surfaces, and processing of physiologically active substances. In relation to the MMPs, there is a report about a transgenic (TG) non-human mammal (mouse) which expresses a human MMP1 (hMMP1) (Non-Patent Document 1). Although this TG mouse expresses hMMP1 in lung tissue, expression in organs of the whole body is not observed. In addition, TG mice which systemically express hMMP2 were not known.

On the other hand, chronic obstructive pulmonary disease (COPD) is a general term configured by unifying the concept of diseases which have been pathologically referred to as emphysema and the concept of diseases which have been clinically referred to as chronic bronchitis. These concepts are clearly described in the international guidelines (GOLD) in 2001 and the clinical practice guidelines of the Japanese Respiratory Society, and these concepts of diseases have been formalized at levels of Japanese and international academic societies. Among the COPDs, emphysema is a disease associated with destructive changes in the alveolar walls, showing a condition that aerated sections ranging from the respiratory tract and the terminal bronchiole to the periphery are abnormally expanded. Although progression of COPD is slow, COPD further progresses to cor pulmonale if left untreated. COPD is a disease that develops through a history of smoking for 20 years or more. In Japan, the number of deaths due to COPD in 2011 was 16, 639, and continues to increase.

### [Prior Art Document]

### [Non-Patent Document]

Non-Patent Document 1: D' Armiento J, Dalal SS, Okada Y, Berg RA, Chada K., Collagenase expression in the lungs of transgenic mice causes pulmonary emphysema, Cell. 1992 Dec 11; 71 (6): 955-61.

### [SUMMARY OF THE INVENTION]

The scope of the invention is as defined in the claims.

### [Problems to be Solved by the Invention]

Systemic expression of hMMP2 in the whole body is required to understand the effects of hMMP2 but such kind of TG non-human mammals has not been so far developed.

In addition, development of model animals preferable for studying treatment and preventive methods of COPD is required, and some research and development have been conducted, but no satisfactory model animals have been developed.

The present invention was made in view of the circumstances described above, and its objective is to provide TG mice which express hMMP2 in tissues of the whole body and to provide a model animal for COPD and other disease models.

### [Means for Solving the Problems]

For accomplishing the above-mentioned objectives, the hMMP2-expressing TG mouse of the present disclosure includes a chicken β-actin promoter for gene expression and a whole gene region of the hMMP2 which is placed on the downstream of the chicken β-actin promoter to induce its systemic expression.

Mice are preferable because easy handling. Furthermore, the strain C57BL/6J is preferable.

Additionally, in order to increase the expression level of hMMP2, a CMV enhancer is placed on the upstream of the promoter region. For the enhancer, not only one enhancer but also a plurality of the same or different enhancers can be used, or otherwise a combination of a plurality of different enhancers can be used, e.g. CMV enhancer, SV40 enhancer, hTERT enhancer, etc., can be used. When using a plurality of different enhancers, their order is not limited. For example, hTERT enhancer, SV40 enhancer and CMV enhancer linked in this order can be shown as an example.

Note that when the expression level of the hMMP2 is excessively high, the average lifespan of the TG mice is likely to become significantly short, and thus an excessive expression should be properly adjusted.

For developing the hMMP2-expressing TG mouse an hMMP2 expression construct is prepared by incorporating the whole gene region of the human matrix metalloproteinase 2 (hMMP2) into the downstream of a CMV enhancer and a chicken β-actin promoter so that the gene can be expressed; this hMMP2 expression construct is introduced into a fertilized egg, suitable for implantation into a mouse for subsequent delivery.

At this time, in some aspects of the disclosure, after the mouse is allowed to deliver, each offspring is raised as an individual founder candidate, genomic DNA is extracted from tissues of each individual founder candidate, and then the presence of the hMMP2 expression construct is confirmed to obtain an individual founder in which the presence of the hMMP2 expression construct can be confirmed.

The TG mouse obtained by the present disclosure characteristically can develop chronic obstructive pulmonary disease (COPD), extrapulmonary lesions (renal failure, liver failure, encapsulating peritoneal sclerosis) and other disease models including emphysema; this TG mouse can be also used to induce chronic obstructive pulmonary disease (COPD), extrapulmonary lesions (renal failure, liver failure, encapsulating peritoneal sclerosis) and other disease models in a short period of time by administering an inducer, and to develop pulmonary fibrosis, airway remodeling, pulmonary hypertension and other disease models. In addition, it is known that decreased muscle mass, osteoporosis, anemia and cardiovascular lesion developed during advanced COPD, and this TG mouse of the present disclosure can also develop these associated disorders. The inducer may include cigarette smoke, a cigarette smoke extract or a cigarette component as well as other substances including albumin, bleomycin, environmental pollutants, protease and others. The administration method of these inducers may include inhalation, intravenous injection, oral administration, intraperitoneal administration, subcutaneous administration, transtracheal administration, etc.

### [Effects of the Invention]

According to the present disclosure, a TG mouse which systemically expresses hMMP2, spontaneoulsy develops COPD extrapulmonary lesions over a long-term basis, and can develop COPD and extrapulmonary lesions in a short period of time by administering cigarette smoke extracts or other inducers; it can also develop pulmonary fibrosis, airway remodeling and pulmonary hypertension by administering specific inducers. Herein, the long-term basis means around 8 to 12 months, and the short period of time means around 2 to 4 weeks. Research on COPD and other diseases can be dramatically advanced by using this hMMP2-expressing TG mouse.

### [BRIEF DESCRIPTION OF THE figures]

Fig. 1 illustrates the strategy for preparation of the hMMP2 expression construct (expression vector).
Fig. 2 illustrates a map of a summary of the hMMP2 expression construct.
Fig. 3 illustrates the confirmation of the base sequences of the CAG expression construct and the hMMP2 cDNA.
Fig. 4 shows a gel photograph with fragments of the hMMP2 expression construct degraded by various restriction enzymes and their electrophoretical migration.
Fig. 5 shows gel photographs performed during purification of the CAG-hMMP2 expression construct. (A) is a photograph showing the CAG-hMMP2 expression construct degraded by *Hind III+Spe I and* electrophoresis migration migrated in a 1% agarose gel, (B) is a photograph showing the electrophoresis migration of the *Hind III*+*Spe I* fragment of the CAG-hMMP2 expression construct in a 0.8% agarose gel, and (C) is a photograph showing the electrophoresis migration of the CAG-hMMP2 that was cut out and separated from the agarose gel for purification using an 1% agarose gel.
Fig. 6 illustrates photographs showing a genomic DNA extracted from the tail tissue of an individual founder candidate, a CAG-hMMP2 expression construct, a positive control containing 1, 3, 10, 30 copies and a control mouse genomic DNA, as well as a negative control DNA containing only the control mouse genomic DNA that were degraded by by the restriction enzyme *EcoR* I and then separated by electrophoresisin an agarose gel.
Fig. 7 shows results of the Southern hybridization using a [³²P]-labeled probe that was carried out for detecting specific signals of the CAG-hMMP2 expression construct.
Fig. 8 shows the results of the Southern hybridization performed using tail tissue DNA of from an individual founder of hMMP2-expressing TG mouse;, the signal intensities of the control signals with known copy numbers were compared.
Fig. 9 shows the expression of the newly inserted hMMP2 gene and that of endogenous mouse Mmp2 gene in various tissues from the hMMP2-expressing TG mouse as evaluated by RT-PCR.
Fig. 10 shows the expressions of the newly inserted hMMP2 gene and endogenous mouse Mmp2 gene in various tissues of the hMMP2-expressing TG mouse and wild-type mouse as investigated by RT-PCR.
Fig. 11 shows normal lung CT images of a wild-type mouse. (A) is a cross-section image in a direction perpendicular to the backbone, and (B) is a cross-section image along a direction of the backbone (The same applies to Fig. 12).
Fig. 12 shows lung CT images of the hMMP2-expressing TG mouse. A low attenuation area pointed by an arrow shows the presence of emphysema.
Fig. 13 shows the number of leukocytes in a bronchoalveolar lavage fluid (BALF)in a mouse which inhaled saline and a hMMP2-expressing TG mouse which inhaled cigarette smoke extract.
Fig. 14 are microphotographs of a wild-type mouse normal lung after staining.
Fig. 15 are microphotographs of the lung from an hMMP2-expressing TG mouse after inhalation of cigarette smoke extracts.
Fig. 16 shows the microphtographs of lungs showing the inhibitory effects of NFκB siRNA on COPD in hMMP2-expressing TG mice.
Fig. 17 shows graphs showing the results of (A) the total cell number in BALF, (B) TNF α concentration , (C) IFN γ concentration , (D) ratio of CD8 T cell number in spleen, (E) spleen CD4/CD8 ratio and (F) TNFα/GAPDH ratio, anda photograph (G) showing the expression of TNF α in samples extracted from each group.
Fig. 18 shows the results obtained after infusion of bleomycin in hMMP2-expressing TG mice. (A) shows a graph of the total cell number in BALF, (B) shows a graph of the total protein concentration in BALF, (C) shows a graph of the MCP-1/GAPDH ratio in lung tissue; (D) is a lung microphotograph of a hMMP2-expressing TG mouse treated with saline and (E) is a lung microphotograph of a hMMP2-expressing TG mouse treated with bleomycin.
Fig. 19 shows results obtained after ovalbumin in hMMP2-expressing TG mice. (A) shows a graph of the total cell number in BALF, (B) shows a graph of the IL5/GAPDH ratio in the lung tissue, (C) shows a graph of IL-4 mRNA expression in lung tissue, (D) shows a graph of the IL-13/GAPDH ratio in lung tissue, (E) is a lung microphotograph of a hMMP2-expressing TG mouse after saline inhalation, and (F) is a lung microphotograph of a hMMP2-expressing TG mouse after ovalbumin inhalation.

### [DESCRIPTION OF THE PREFERRED EMBODIMENTS]

Next, embodiments of the present disclosure will be explained with reference to figures and tables.

### <Preparation of hMMP2-expressing TG Mouse>

### 1. Construction of hMMP2 expression vector

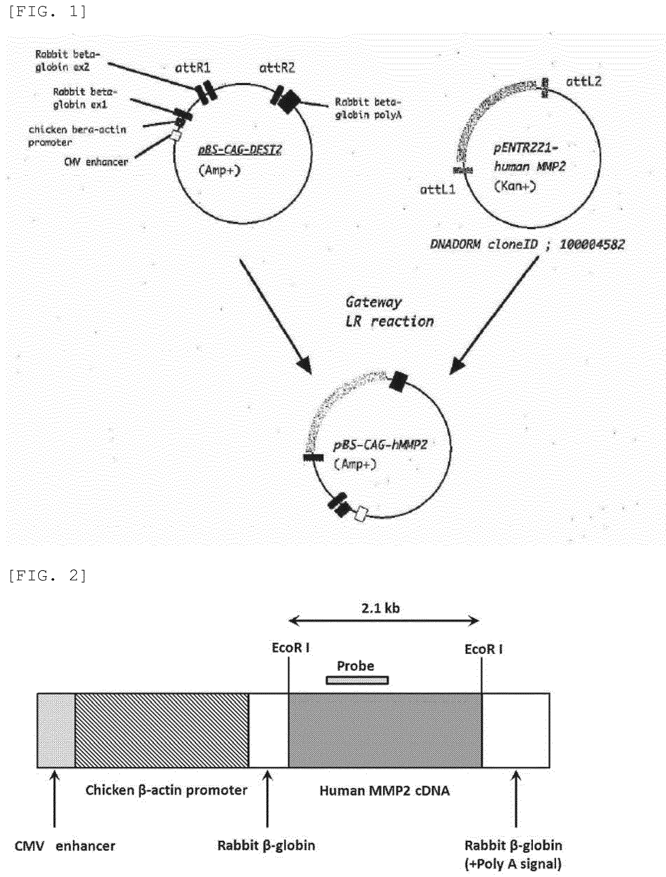

As shown in Fig. 1, an hMMP2 expression vector pBS-CAG-hMMP2 was constructed by subcloning an hMMP2 cDNA fragment into a pBS-CAG-DESTZ vector, from a pENTR vector (pENTR221-human MMP2) into which the hMMP2 cDNA (accession number: BC002576) was cloned, by using Gateway LR reaction (Invitrogen) (Fig. 1 and Fig. 2). The hMMP2 expression construct was set so as to have only one *EcoRI* site for genetic analysis of TG mice.

The hMMP2 cDNA was expressed by an early-immediate enhancer of human cytomegalovirus (CMV enhancer), followed by a chicken β actin in which a promoter, a first exon and an intron of a chicken β actin were linked (chicken β-actin promoter). A transcript of hMMP2 having a stop codon and a poly (A) signal was linked to an upstream of a poly (A) sequence (poly A signal) in a rabbit β-globin (Figure 2). Although the hMMP2 is represented by "human MMP2" or "Human MMP2" in Fig. 1 and Fig. 2, they are used as symbols referring to the same subject matter.

An mRNA transcript of the transgene is constructed as a part of a first exon (which is transcribed but not translated) of the chicken β-actin, to which the hMMP2 cDNA is transcribed.

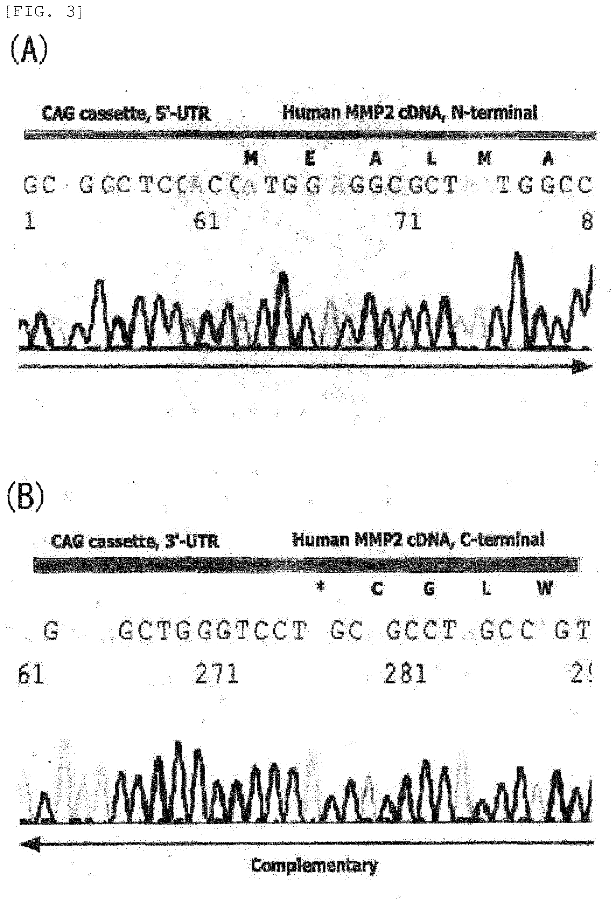

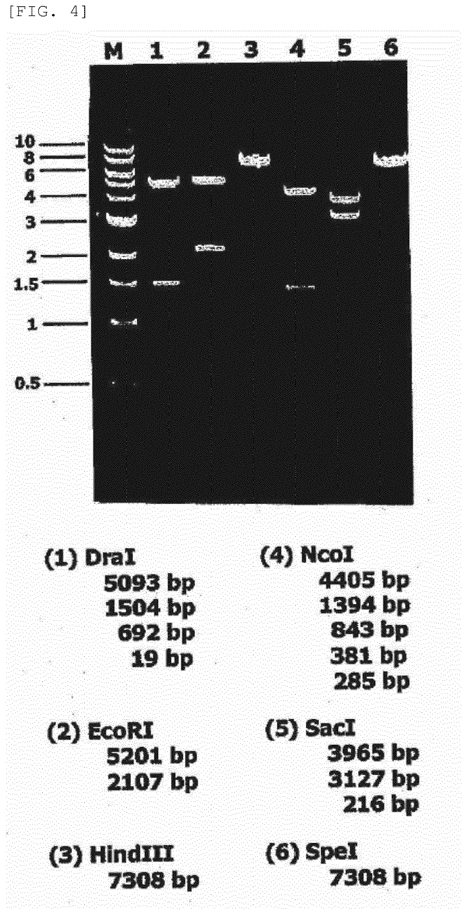

After the hMMP2 expression construct was constructed, the DNA sequence on the site bound to the hMMP2 cDNA was confirmed by sequence analysis and restriction enzyme mapping. As a result, it was confirmed that the hMMP2 cDNA had been cloned into a CAG expression vector as planned (Fig. 3). In addition, sizes of fragments made by restriction enzymes *Dra I, EcoR I, Hind III, Nco I, Sac I, Spe I* were consistent with the expected fragment sizes (Fig. 4).

With the above results, construction of the hMMP2 expression construct was completed.

### 2. Purification of a linear DNA for producing TG mouse

After the expression vector pBS-CAG-hMMP2 of the hMMP2 was introduced into a DH5α competent cell (Invitrogen), the cell was seeded on an LB agar medium containing ampicillin, and an ampicillin-resistant strain was selected. A single colony of the ampicillin-resistant strain was picked up, and cultured while shaking in a liquid medium all day and night.

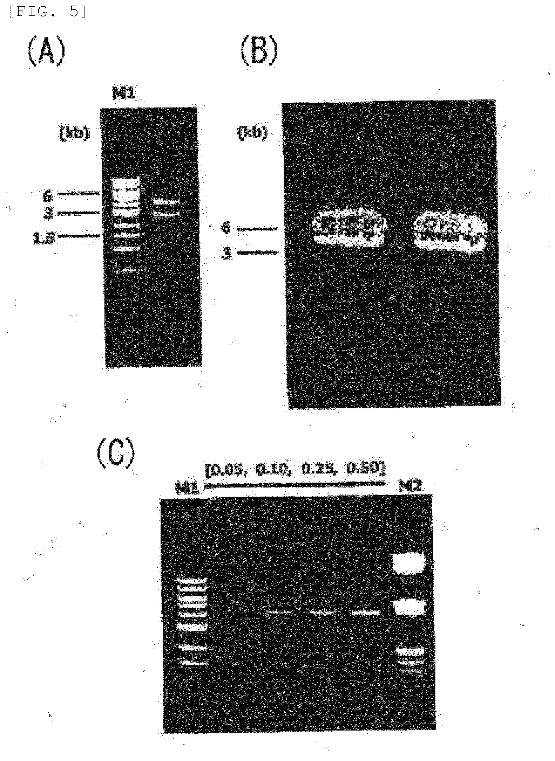

The cloned pBS-CAG-hMMP2 was purified by a plasmid extraction kit (Plasmid Midi kit, QIAGEN), to which restriction enzymes *Hind III* + *Spe I* were added, and incubated at 37°C for 16 hours. Productions of a DNA fragment derived from the vector and a DNA fragment for hMMP2 expression were confirmed by 1% agarose gel electrophoresis (FIG. 5 (A)). Subsequently, the DNA fragments were collected by phenol/chloroform extraction and isopropanol precipitation.

The collected DNA fragments were redissolved in TE, electrophoresed in a 0.8% agarose gel, and then a DNA fragment for hMMP2 expression was excised from the separated DNA fragments. The excised DNA fragment was purified by a DNA extraction kit (DNA gel extraction kit, Qiagen), its purity was confirmed by 1% agarose gel electrophoresis, and its concentration was determined by NanoDrop spectrophotometer (AGC TECHNO GLASS Co., Ltd.). The DNA fragment was diluted so that its concentration was 2ng/µL to obtain a DNA solution of hMMP2 (CAG-hMMP2 expression construct) for microinjection. This solution was stored at -25°C until use in the test.

### 3. Microinjection of expression construct into fertilized egg

Fertilized eggs were sampled from a female mouse which had been subjected to superovulation induction by administering PMSG and hCG, and the CAG-hMMP2 expression construct was introduced into the eggs by a microinjection method. The fertilized egg into which the CAG-hMMP2 expression construct had been introduced was implanted into the fallopian tube of a pseudopregnant mouse.

From C57BL/6J female mice crossed after superovulation induction, 862 fertilized eggs were sampled. Among them, 266 fertilized eggs were injected with the CAG-hMMP2 expression construct. When the fertilized eggs after injection were microscopically observed, 236 fertilized eggs were in stable states even after the microinjection. Among them, 210 fertilized eggs could be implanted into pseudopregnant mice.

### 4. Raising of hMMP2-expressing TG mouse and confirmation of individual founder

### (1) Raising of hMMP2-expressing TG mouse

Offspring obtained through spontaneous delivery from fertilized eggs of C57BL/6J mice microinjected with the CAG-hMMP2 expression construct were raised until weaning. The hMMP2 TG mouse individual founder candidates were weaned at 3 weeks old, attached with ear tags for fixing identifications, then their tail tissues were subjected to biopsy and stored at -80°C until analysis.

210 fertilized eggs were implanted, and after an about 3-week of pregnancy period, 65 mouse offspring could be obtained from surrogate mice into which fertilized eggs injected with the CAG-hMMP2 expression construct were implanted. The surrogate mice, from which these offsprings were born, raised them, and as a result all of 65 individuals could be raised until weaning. From all of these weaned individuals and a part of individuals dead after weaning, tail tissue samples were collected.

The number of offsprings from the early embryos into which the expression construct had been introduced and the number of weaned individuals were preferable. Consequently, it was considered that there was no adverse effect from the injection of the expression construct for development and differentiation of the fertilized eggs.

### (2) Genotyping of individual founder of hMMP2 expression TG mouse.

The tail tissue of the individual hMMP2 TG mouse stored at -80°C was melted at room temperature, a buffer solution for dissolution containing 1% of SDS (Wako Pure Chemical Industries, Ltd.), 1 mg/mL of Actinase E (Kaken Pharmaceutical Co., Ltd.) and 0.15 mg/mL of Protease K (Merck KGaA) was added, and shaken at 55°C for 16 hours to solubilize the tissues. Proteins which bind to the solubilized genomic DNA were removed from the tissues by phenol extraction and phenol/chloroform extraction. The RNA contained in the genomic DNA was decomposed by RNaseA (Sigma), and then high-molecular genomic DNA was precipitated by isopropanol precipitation. The genomic DNA was washed with 70% ethanol and air-dried, and then redissolved in 50 µL of TE.

A DNA concentration of the genomic DNA solution prepared from each sample was determined by spectrophotometry, and the volume of the genomic DNA solution corresponding to 5µg of DNA was determined from the DNA concentration value of each sample.

The CAG-hMMP2 expression construct used for microinjection was diluted so that the copy number for each diluted solution was 1, 3, 10 or 30 copies, to which 5 µg of a separately prepared control mouse genomic DNA was added to prepare a positive control DNA for Southern blotting. On the other hand, 5 µg of genomic DNA of the control mouse was used as a negative control DNA for Southern blotting.

The concentration of the genomic DNA prepared by extraction from the tissue of the individual hMMP2 TG mouse founder that have been raised up to weaning was sufficient for Southern analysis (5 µg of DNA) (Table 1).

**[Table 1]**

| (ng/*µ*l) | | | | |
|---|---|---|---|---|
| Sample No. | ID | Sex | Date of Birth | Genomic DNA conc |
| 1 | 1_1 | ♂ | 10/12/20 | 473 |
| 2 | 1_2 | ♂ | 10/12/20 | 664 |
| 3 | 1_3 | ♂ | 10/12/20 | 280 |
| 4 | 1_4 | ♂ | 10/12/20 | 443 |
| 5 | 1_5 | ♂ | 10/12/20 | 749 |
| 6 | 1_6 | ♂ | 10/12/20 | 182 |
| 7 | 1_7 | ♂ | 10/12/20 | 668 |
| 8 | 1_8 | ♂ | 10/12/20 | 720 |
| 9 | 1_9 | ♂ | 10/12/20 | 780 |
| 10 | 1_10 | ♂ | 10/12/20 | 504 |
| 11 | 2_1 | ♂ | 10/12/20 | 484 |
| 12 | 2_2 | ♂ | 10/12/20 | 568 |
| 13 | 2_3 | ♂ | 10/12/20 | 674 |
| 14 | 2_4 | ♂ | 10/12/20 | 556 |
| 15 | 2_6 | ♂ | 10/12/20 | 883 |
| 16 | 2_6 | ♂ | 10/12/20 | 914 |
| 17 | 2_7 | ♂ | 10/12/20 | 483 |
| 18 | 2_8 | ♂ | 10/12/20 | 636 |
| 19 | 2_9 | ♂ | 10/12/20 | 777 |
| 20 | 2_20 | ♂ | 10/12/20 | 845 |
| 21 | 3_1 | ♂ | 10/12/20 | 737 |
| 22 | 3_2 | ♂ | 10/12/20 | 418 |
| 23 | 3_3 | ♂ | 10/12/20 | 929 |
| 24 | 3_4 | ♂ | 10/12/20 | 913 |
| 25 | 3_5 | ♂ | 10/12/20 | 735 |
| 26 | 3_6 | ♂ | 10/12/20 | 478 |
| 27 | 3_7 | ♂ | 10/12/20 | 813 |
| 28 | 3_8 | ♂ | 10/12/20 | 1021 |
| 29 | 3_9 | ♂ | 10/12/20 | 761 |
| 30 | 3_30 | ♂ | 10/12/20 | 632 |
| 31 | 4_1 | ♀ | 10/12/20 | 741 |
| 32 | 4_2 | ♀ | 10/12/20 | 774 |
| 33 | 4_3 | ♀ | 10/12/20 | 845 |
| 34 | 4_4 | ♀ | 10/12/20 | 1042 |
| 35 | 4_5 | ♀ | 10/12/20 | 946 |
| 36 | 4_6 | ♀ | 10/12/20 | 843 |
| 37 | 4_7 | ♀ | 10/12/20 | 605 |
| 38 | 4_8 | ♀ | 10/12/20 | 592 |
| 39 | 4_9 | ♀ | 10/12/20 | 1013 |
| 40 | 4_40 | ♀ | 10/12/20 | 403 |
| 41 | 5_1 | ♀ | 10/12/20 | 573 |
| 42 | 5_2 | ♀ | 10/12/20 | 851 |
| 43 | 5_3 | ♀ | 10/12/20 | 641 |
| 44 | 5_4 | ♀ | 10/12/20 | 783 |
| 45 | 5_5 | ♀ | 10/12/20 | 645 |
| 46 | 5_6 | ♀ | 10/12/20 | 893 |
| 47 | 5_7 | ♀ | 10/12/20 | 970 |
| 48 | 5_8 | ♀ | 10/12/20 | 902 |
| 49 | 5_9 | ♀ | 10/12/20 | 403 |
| 50 | 5_50 | ♀ | 10/12/20 | 700 |
| 51 | 6_1 | ♀ | 10/12/20 | 675 |
| 52 | 6_2 | ♀ | 10/12/20 | 638 |
| 53 | 6_3 | ♀ | 10/12/20 | 671 |
| 54 | 6_4 | ♀ | 10/12/20 | 617 |
| 55 | 6_5 | ♀ | 10/12/20 | 885 |
| 56 | 6_6 | ♀ | 10/12/20 | 685 |
| 57 | 6_7 | ♂ | 10/12/20 | 984 |
| 58 | 7_1 | ♂ | 10/12/20 | 714 |
| 59 | 7_2 | ♂ | 10/12/20 | 629 |
| 60 | 7_3 | ♂ | 10/12/20 | 500 |
| 61 | 7_4 | ♂ | 10/12/20 | 640 |
| 62 | 7_5 | ♂ | 10/12/20 | 1160 |
| 63 | 7_6 | ♂ | 10/12/20 | 754 |
| 64 | 7_7 | ♂ | 10/12/20 | 806 |
| 65 | 7_8 | ♂ | 10/12/20 | 1031 |

A restriction enzyme *EcoR I* was added to the genomic DNA prepared from each sample, the positive control DNA and the negative control DNA, and incubated at 37°C for 16 hours. The *EcoR I* fragments of the produced genomic DNA were precipitated by isopropanol precipitation, washed with 70% ethanol, air-dried, and then redissolved in TE. These genomic DNA fragments were electrophoresed using a 1.2% agarose gel, the genomic DNA fragments separated in the agarose gel were visualized by a UV transilluminator and photographed together with a scale.

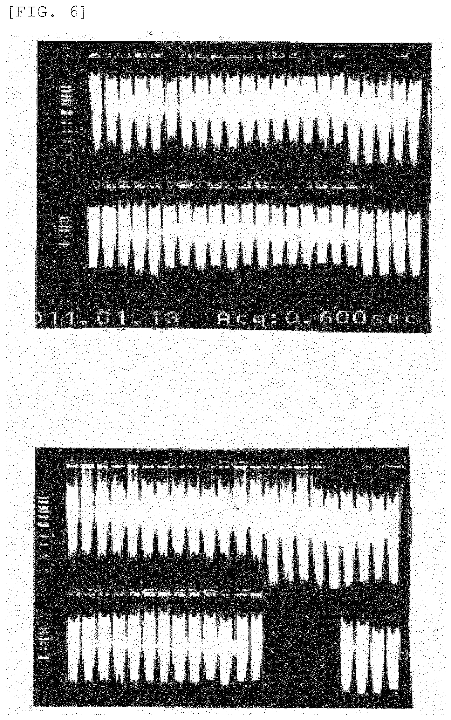

As shown in FIG. 6, high-molecular to low-molecular DNA fragments were observed in the agarose gel after electrophoresis, and various sizes of produced DNA fragments were observed to be uniformly separated by electrophoresis.

This agarose gel was immersed in 0.25 N hydrochloric acid, gently shaken for 10 minutes, then immersed in 0.4 N sodium hydroxide, and furthermore gently shaken for 10 minutes. The genomic DNA fragment separated in the agarose gel was transferred to a nylon membrane (Hybond-XL; GEH) by a capillary method using 0.4 N sodium hydroxide at room temperature for 16 hours. The nylon membrane to which the genomic DNA fragments were transferred was immersed in 2×SSC, gently shaken for 10 minutes, then air-dried, and stored at room temperature until being used for the hybridization.

The hMMP2 probe 2 fragment was labeled with [³²P] using a DNA labeling kit (Megaprime DNA Labelling System; GEH) by a random prime method. A [³²P]-labeled fragment was produced using Sephadex spin column (ProbeQuant G-50 Micro Columns; GEH), and referred to as [³²P]-labeled hMMP2 probe 2.

The nylon membrane to which the genomic DNA fragments were transferred was put in a hybridization buffer solution and preincubated at 65°C for 1 hour. Subsequently, it was heated at 95°C for 5 minutes, and thereafter cooled in ice for 5 minutes, to which the denatured [³²P] -labeled hMM2 probe was added, and incubated at 65°C for 4 hours. After that, the nylon membrane was taken out, and washed with 0.1% SDS and 0.5×SSC at 65°C for about 15 minutes. Radioactivity originating in the probe bound to the membrane was monitored by a radiation survey meter, and washed repeatedly until the radioactivity was nearly constant.

The membrane after washing was covered with a wrap film, lapped with an X-ray film (BioMax MS; Kodak) in a darkroom, and then put in an autoradiography cassette. After being exposed to light at 4°C for 1 week, the X-ray film was developed. Specific signals of 2.1 kb originating in the CAG-hMMP2 expression construct were detected by autoradiography, and mice showing specific signals after hybridization with the [³²P] -labeled probe were identified as hMMP2 TG mouse founder individuals. The signal intensity of each individual was compared to the signal intensity of the positive control DNA, to estimate the copy number of CAG-hMMP2 expression construct introduced into the genome.

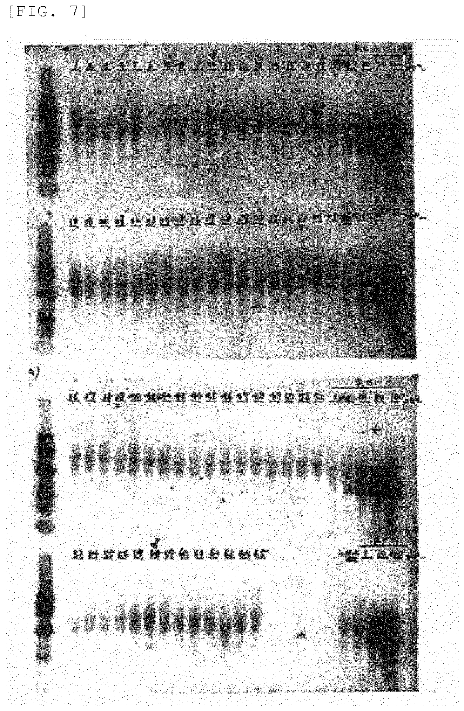

As shown in FIG 7, specific signals originating in the expression construct could be detected from the fragments of the CAG-hMMP2 expression constructs which are all positive controls, by hybridization using the [³²P]-labeled probe. Since the hybridization signals originating in the expression constructs could be detected from all positive controls, the Southern analysis using the [³²P]-labeled probe indicated that the expression constructs having one or more copies introduced into the host genome could be detected.

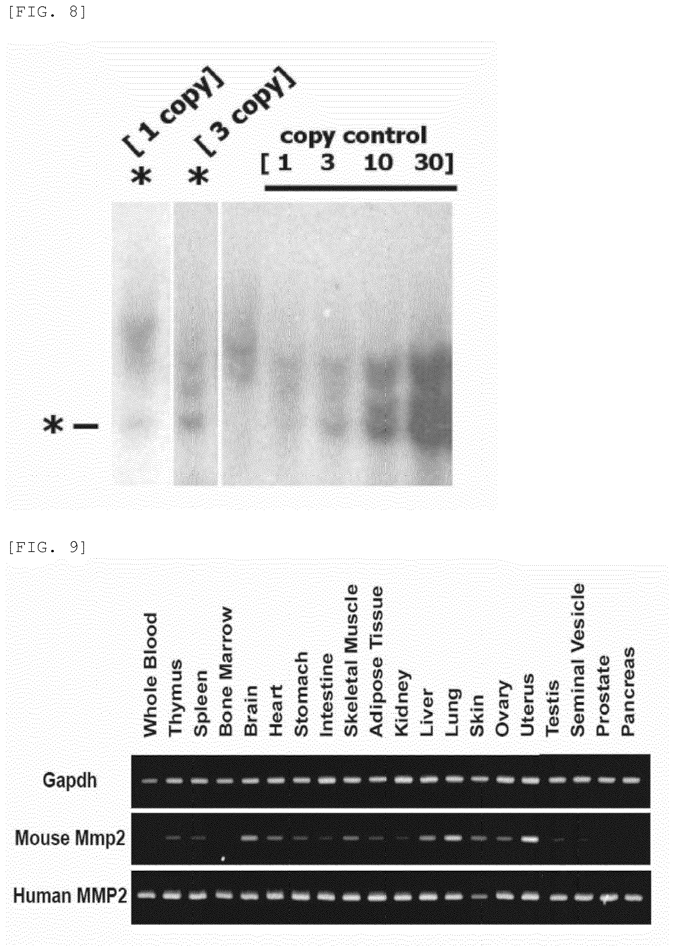

Further, as shown in FIG. 8, transgenes were confirmed in 2 mice (3%) out of 65 founder candidate individuals by Southern blotting using tail DNA. Copy numbers of the expression constructs introduced into these transgenic mouse founders were 1 to 3 copies.

### <hMMP2 Expression in various tissues of hMMP2-expressing TG Mouse>

RNA was purified from various tissues of mice by Trizol reagent (Invitrogen) following manual instruction. The RNA sample was reverse-transcribed using oligo dT by SuperScript (Invitrogen) to obtain DNA. Using a PTC-100 thermal controller (MJ Research), PCR reaction was carried out in 28 cycles (for GAPDH) or 38 cycles (for human MMP2 and mouse Mmp2), the one cycle including 10 seconds at 94°C, 20 seconds at 60°C and 40 seconds at 72°C, and finally extension reaction was carried out at 72°C for 5 minutes . The control reaction was carried out for the RNA samples which were not reverse-transcribed. The primers used for amplification of GAPDH, mouse Mmp2 and human MMP2 gene were as follows. For mGapdh, 5'-CCCTTATTGACCTCAACTACATGGT-3' (SEQ ID NO: 1) as a sense primer and 5'-GAGGGGCCATCCACAGTCTTCTG-3' (SEQ ID NO: 2) as an antisense primer were used, for mMmp2, 5'-CACCACCGAGGACTATGACC-3' (SEQ ID NO: 3) as a sense primer and 5'-TGTTGCCCAGGAAAGTGAAG-3' (SEQ ID NO: 4) as an antisense primer were used, and for hMMP2, 5'-TACTGGATCTACTCAGCCAGCAC-3' (SEQ ID NO: 5) as a sense primer and 5'-CAGGATCCATTTTCTTCTTCACC-3' (SEQ ID NO: 6) as an antisense primer were used.

Fig. 9 shows expressions of the exogenous hMMP2 gene and the endogenous mouse Mmp2 gene as investigated by RT-PCR in various tissues of the hMMP2-expressing TG mice (whole blood, thymus, spleen, bone marrow, brain, heart, stomach, intestine, skeletal muscle, adipose tissue, kidney, liver, lung, skin, ovary, uterus, testis, seminal vesicle, prostate and pancreas). Gapdh was used as a positive control. Although the expression of the endogenous mouse Mmp2 was observed in a lot of tissues, there were wide differences in the expression levels among the tissues. Also, there were tissues (whole blood, bone marrow, seminal vesicle, prostate and pancreas) in which no band of the mouse Mmp2 was confirmed. On the other hand, the hMMP2 showed a high level of expression in all evaluated tissues.

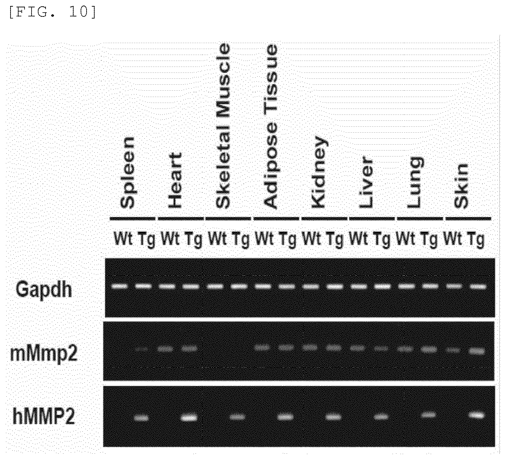

Fig. 10 shows expressions of the hMMP2 gene and the mMmp2 gene as examined by RT-PCR in various tissues (spleen, heart, skeletal muscle, adipose tissue, kidney, liver, lung, skin) of the hMMP2-expressing TGmice and wild-type mice. Expression of the hMMP2 gene was observed in all tissues of the TG mice, but it was not observed in wild-type mice. There was no difference in expression of the mMmp2 gene, between the tissues of the TG mice and the wild-type mice. In Fig. 9, the mMmp2 and the Mouse Mmp2 are used as symbols referring to the same subject matter.

### <Findings by Computed Tomography>

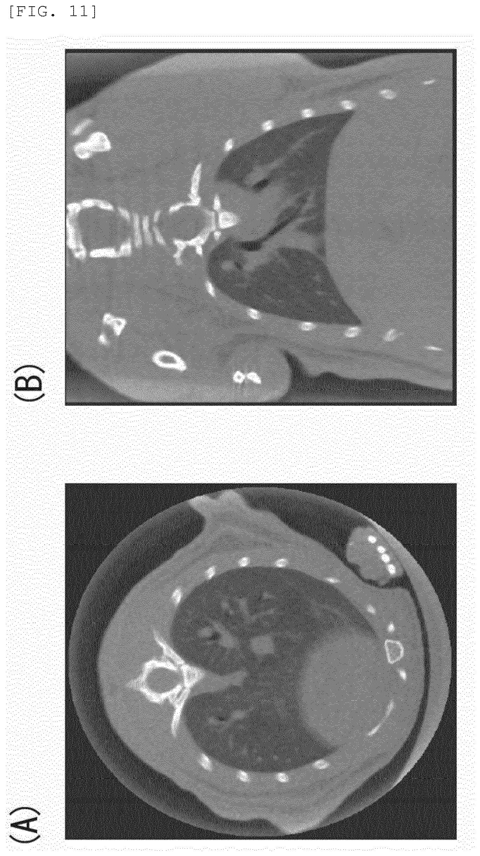

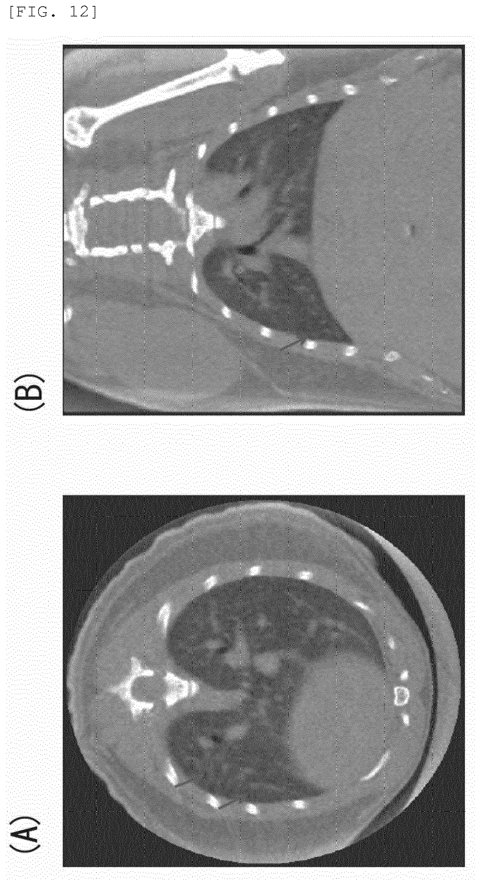

The lungs of a wild-type mouse and an hMMP2-expressing TG mouse were observed by computer tomography (CT). Fig. 11 shows a CT image of a normal lung of the wild-type mouse. Fig. 12 shows a CT image of a lung of the hMMP2-expressing TG mouse. The low attenuation field (region indicated by an arrow) indicates emphysema.

### <Severe Inflammation Changes and Chronic Obstructive Pulmonary Disease in hMMP2-expressing TG Mouse after Inhalation of Cigarette Smoke Extract>

The hMMP2-expressing TG mice were subjected to inhalation of cigarette smoke extract and saline, and then leukocyte numbers in the bronchoalveolar lavage fluid (BALF) were counted.

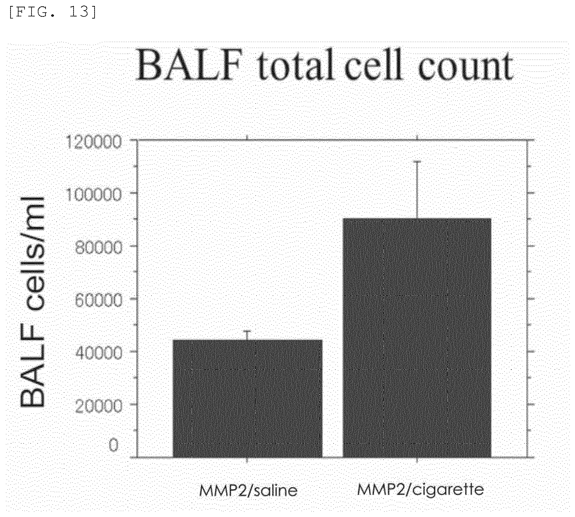

As shown in Fig. 13, the hMMP2-expressing TG mice which inhaled cigarette smoke extract (MMP2/cigarette) showed an increased number of leukocytes in BALF compared to the hMMP2-expressing TG mice which inhaled saline (MMP2/saline), indicating that the TG mice have exacerbated inflammation.

### <Histological Findings of the Lungs>

The histological findings of the lungs of the wild-type mice and the hMMP2-expressing TG mice which inhaled cigarette smoke extract were evaluated. The lung tissues of the respective mice were stained with hematoxilin/eosin, and microscopically observed.

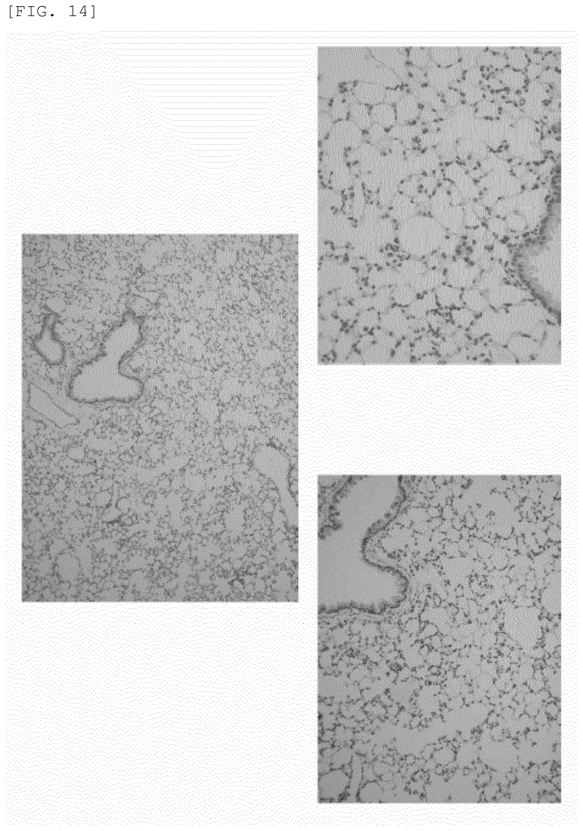

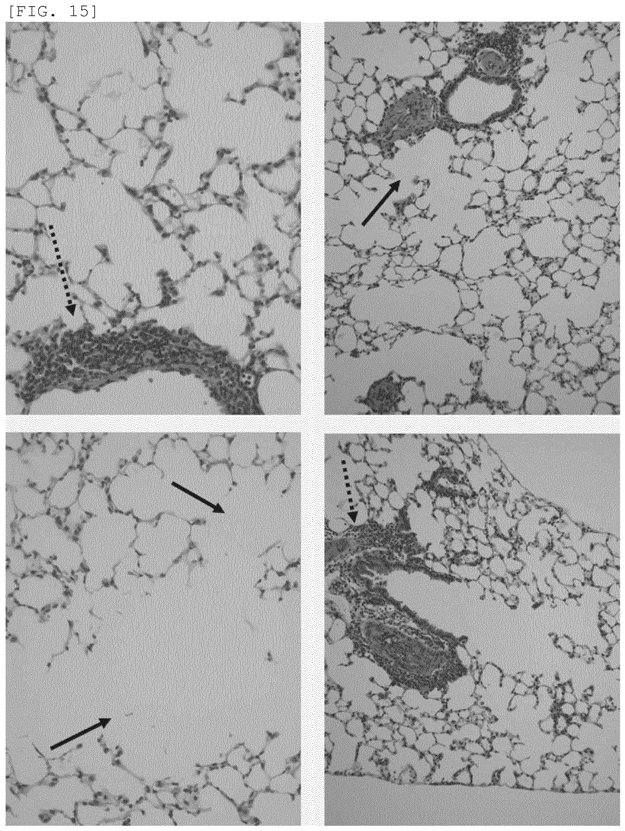

Fig. 14 shows microphotographs of the normal lung in the wild-type mouse. Fig 15 shows the hMMP2-expressing TG mouse lung after inhalation of cigarette smoke extract. In the hMMP2-expressing TG mouse lung, exacerbation of chronic obstructive pulmonary disease (COPD) was observed (solidarrow), and infiltration of leucocytes was increased around the blood vessels and the bronchus (dashed arrow).

### <Inhibitory Effect of NF_{K}B siRNA in COPD Using hMMP2 Expression TG Mice>

Development of COPD requires production of inflammatory cells and inflammatory cytokines such as TNF-α, IL-1β and IL-6 that result from activation of lung tissue constituent cells, and the pathway of phosphatidylinositol-3-kinase (PI3)-protein kinase C-nuclear factor-κB (NFκB) plays an important role. Thus, we focused on the intracellular signaling mechanism of the NF_{K}B pathway, and the effect of the inhalation of NF_{K}B siRNA on the development of COPD was studied.

### 1. Test method

Mice were divided into 5 groups and set as follows. That is, they were divided into (A) group in which the wild mice were subjected to saline inhalation (wild-type mice + saline; n=6), (B) group in which the hMMP 2-expressing TG mice were sub jected to saline inhalation (MMP-2 mice + saline; n=5), (C) group in which the wild-type mice were subjected to inhalation of cigarette smoke extract (wild-type mice + cigarette smoke extract; n=10), (D) group in which the hMMP2-expressing TG mice were subjected to inhalation of cigarette smoke extract (MMP-2 mice + cigarette smoke extract; n=10), and (E) group in which the hMMP2-expressing TG mice were subjected to inhalation of cigarette smoke extract and intranasally treated with NFκβ siRNA (MMP-2 mice + cigarette smoke extract + NF_{K}B siRNA; n=3) .

### (1) Production of smoking-induced COPD mouse model

The hMMP2-expressing TG mice were exposed to cigarette smoke extract for 60 minutes every day for two weeks to produce an emphysema model. As a control, C57BL/6 wild-type mice were used.

### (2) Nucleic acid administration method

NF_{K}B siRNA was dissolved with distilled water and intranasally administered, prior to exposure to the cigarette smoke extract and at the 0th, 2nd, 4th, 6th, 8th, 10th and 12th day of the exposure.

### (3) RT-PCR

The mRNA expression was studied by RT-PCR method. Total RNA was isolated from a lung tissue by treatment with TRIZOL (Invitrogen, Carlsbad, CA). The single-strand cDNA was synthesized using a reverse transcriptase (Invitrogen) and oligo (dT). For amplification of the desired cDNA, using AB Applied Biosystem 7600 and Gold AmpliTaq (AB Applied Biosystem, Foster City, CA) and a primer specific to the desired cDNA, reaction was carried out in a reaction solution at 94°C for 10 minutes, and then followed by an optimal number of cycles, the one cycle including 30 seconds at 94°C, 30 seconds at 55°C and 30 seconds at 72°C. After 2% agarose electrophoresis, the PCR product was colored with ethidium bromide. Density analysis was carried out by an NIH imaging system, an expression level of Glyceraldehyde-3-phosphate dehydrogenase (GAPDH) was set as a standard, and mRNA expressions of cytokines and other factors were studied and compared.

### (4) Measurement and biochemical examination of the cell number

The total cell number in the bronchoalveolar lavage fluid (BALF) was measured using a ChemoMetec (Allerod, Denmark) Nucleocounter.

### (5) The data were statistically investigated by ANOVA.

### 2. Test results

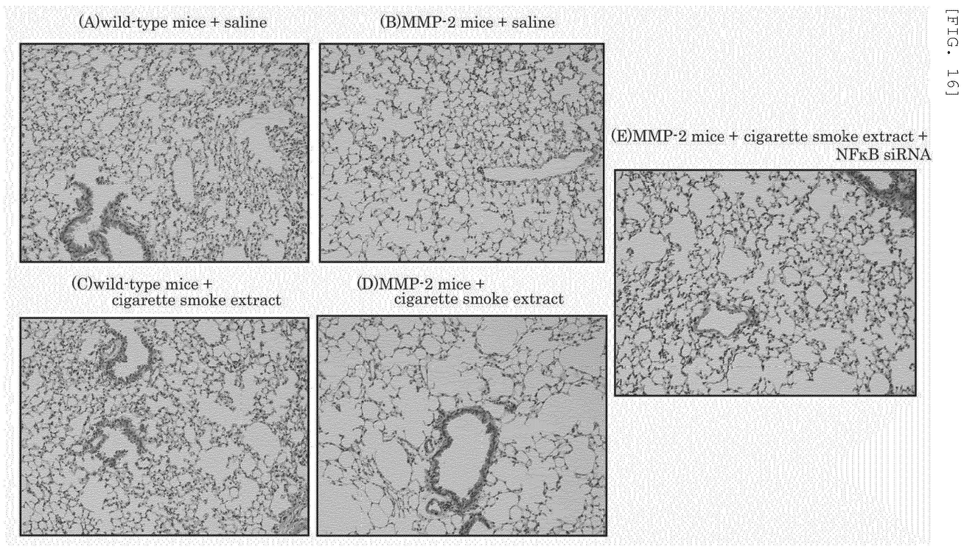

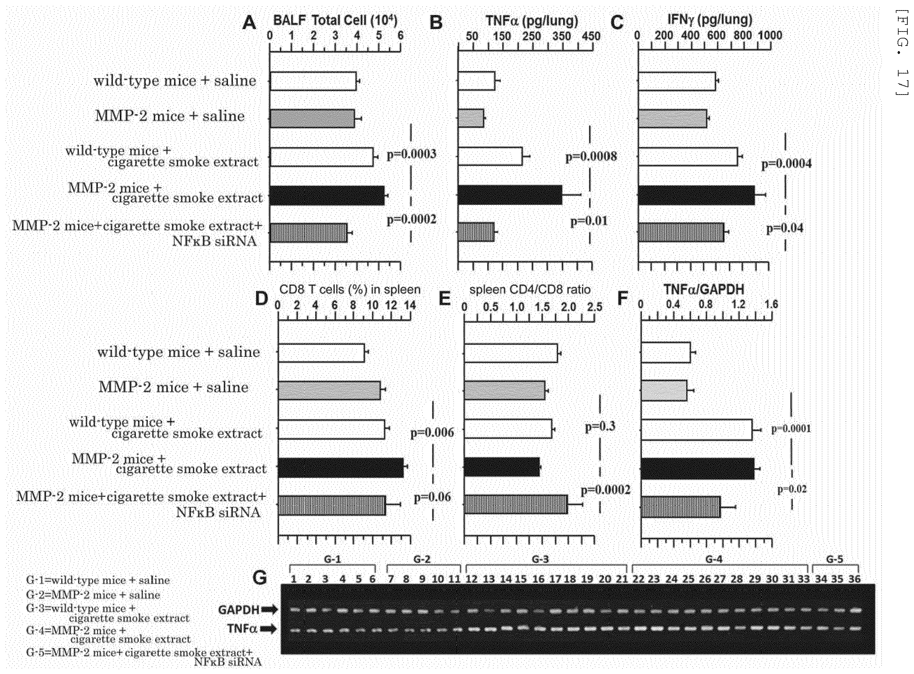

The results are shown in Fig. 16 and Fig. 17.

As shown in Fig. 17, in the group in which the cigarette smoke extract was administered to the hMMP2-expressing TG mice (MMP-2 mice + the cigarette smoke extract), the total cell number in BALF, TNF α, IFN γ and spleen CD8T cells were significantly increased compared to the non-administered group of the hMMP2-expressing TG mice (MMP-2 mice + saline) and the group in which the cigarette smoke extract was administered to the wild-type mice (wild-type mice + cigarette smoke extract). In addition, the spleen CD4/CD8 was significantly decreased. On the other hand, in the group in which the cigarette smoke extract and the NFκB siRNA were administered to the hMMP2-expressing TG mice (MMP-2 mice + cigarette smoke extract + NF_{K}B siRNA), these values were significantly decreased, and the spleen CD4/CD8 were recovered. Note that Fig. 17 (F) is prepared by quantifying the densities of the respective bands in Fig. 17 (G).

As shown in Fig. 16, when comparing the control group ((C) wild-type mice + cigarette smoke extract) and the group in which the cigarette smoke extract was administered to the hMMP2-expressing TG mice ((D) MMP-2 mice + cigarette smoke extract) which showed the same mRNA expression level of TNFα associated with inflammation in Fig. 17 (F), the group in which the cigarette smoke extract was administered to the hMMP2-expressing TG mice ((D) MMP-2 mice + cigarette smoke extract) showed large-scale destruction of the alveolar wall, indicating significant exacerbation of COPD. On the other hand, compared to the group in which the cigarette smoke extract was administered to the hMMP2-expressing TG mouse ((D) MMP-2 mice + cigarette smoke extract), the group in which the cigarette smoke extract and NF_{K}B siRNA were administered to the hMMP2-expressing TG mouse ((E) MMP-2 mice + cigarette smoke extract + NF_{K}B siRNA) showed reduced destruction of the alveolar wall, indicating that changes in emphysema and COPD were significantly decreased.

As described above, the hMMP2-expressing TG mouse group was found to frequently develop COPD by inhalation of cigarette smoke extract.

### <Effect of Bleomycin on hMMP2-expressing TG Mouse>

Saline or bleomycin was administered to hMMP2-expressing TG mice by using an osmotic pump to investigate the effect of bleomycin. As test groups, two groups, a group receiving saline (MMP-2 mice + saline, n=4) and a group receiving bleomycin (MMP-2 mice + bleomycin, n=4) were used.

In order to administer bleomycin, pentobarbital was intraperitoneally injected to the hMMP2-expressing TG mouse (female, 8 weeks old), and then an ALZET osmotic pump was subcutaneously implanted in the back of the mouse. Previously 200 µL of bleomycin or a saline aqueous solution was injected in the ALZET osmotic pump.

On the 21st day after the implantation of the osmotic pumps, pentobarbital was intraperitoneally administered to the mouse, the neck skin and the muscle of the mouse were stripped under anesthesia to expose the trachea. Saline was injected into the trachea using an indwelling needle to collect the bronchoalveolar lavage fluid (BALF) . Subsequently, the thorax was opened, perfused with saline, and the lung tissue was excised.

The mRNA expression was studied by an RT-PCR method. Total RNA was isolated from a lung tissue by treatment with TRIZOL (Invitrogen, Carlsbad, CA). The single-strand cDNA was synthesized using a reverse transcriptase (Invitrogen) and oligo (dT). For amplification of the desired cDNA, using AB Applied Biosystem 7600 and Gold AmpliTaq (AB Applied Biosystem, Foster City, CA) and a primer specific to the desired cDNA, reaction was carried out in a reaction solution at 94°C for 10 minutes, and then followed by an optimal number of cycles, the one cycle including 30 seconds at 94°C, 30 seconds at 55°C and 30 seconds at 72°C. After 2% agarose electrophoresis, the PCR product was stained with ethidium bromide. Density analysis was carried out by an NIH imaging system, an expression level of Glyceraldehyde-3-phosphate dehydrogenase (GAPDH) was set as a standard, and mRNA expressions of cytokines and other factors were studied and compared.

The total cell number in the bronchoalveolar lavage fluid was measured using a ChemoMetec (Allerod, Denmark) Nucleocounter. The total protein amount in the bronchoalveolar lavage fluid was measured by Dye-binding assay (Bio-Rad Laboratories, Hercules, CA). Furthermore, the data were statistically evaluated by ANOVA.

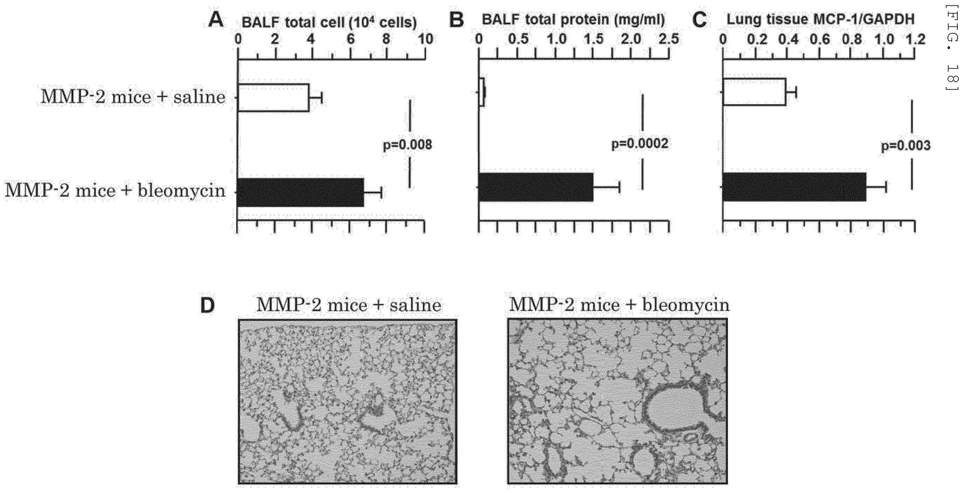

The results are shown in Fig. 18. The group receiving bleomycin (MMP-2 mice + bleomycin) showed significantly high values in the total cell number in BALF (A), the total protein level in BALF (B) and the monocyte chemoattractant protein-1 in the lung tissue (MCP-1) (C) compared to the control group receiving saline (MMP-2 mice + saline). In addition, the group receiving bleomycin showed a significantly high emphysematous changes (D) compared to the control group.

As mentioned above, the hMMP2-expressing TG mouse group had large-scale destruction of the alveolar wall after administration of bleomycin, indicating frequent developments of COPD.

### <Effects of Ovalbumin on hMMP2-expressing TG Mouse>

The hMMP2-expressing TG mice were sensitized with ovalbumin by intraperitoneal administration, then subjected to ovalbumin inhalation through a nebulizer for 5 days to produce asthma models. Control mice were given saline. As test groups, two groups, a group receiving saline (MMP-2 mice + saline, n=3) and a group receiving ovalbumin by intraperitoneal and inhalation administrations (MMP-2 Mice + ovalbumin, n=3) were used.

On the 0th, 7th, 14th and 21st day from the start of the test, 10 µg of ovalbumin and 1 mg of Al(OH)₃, or alternatively saline were intraperitoneally administered, and then on the 28th, 29th, 30th, 31st and 32nd day, the mice were subjected to inhalation of 2% ovalbumin or saline. On the 33rd day from the start of the test, pentobarbital was intraperitoneally administered to the mice, the neck skin and the muscle of the mouse were stripped under anesthesia to expose the trachea. Saline was injected into the trachea using an indwelling needle to collect the bronchoalveolar lavage fluid (BALF). Subsequently, the thorax was opened, perfused with saline, and the lung tissue was excised.

The mRNA expression was studied by RT-PCR. Total RNA was isolated from the lung tissue by treatment with TRIZOL (Invitrogen, Carlsbad, CA). The single-strand cDNA was synthesized using a reverse transcriptase (Invitrogen) and oligo (dT). For amplification of the desired cDNA, using AB Applied Biosystem 7600 and Gold AmpliTaq (AB Applied Biosystem, Foster City, CA) and a primer specific to the desired cDNA, reaction was carried out in a reaction solution at 94°C for 10 minutes, and then followed by an optimal number of cycles, the one cycle including 30 seconds at 94°C, 30 seconds at 55°C and 30 seconds at 72°C. After 2% agarose electrophoresis, the PCR product was stained with ethidium bromide. Density analysis was carried out by an NIH imaging system, an expression level of Glyceraldehyde-3-phosphate dehydrogenase (GAPDH) was set as a standard, and mRNA expressions of cytokines and other factors were studied and compared.

The total cell number in the bronchoalveolar lavage fluid was measured using a ChemoMetec (Allerod, Denmark) Nucleocounter. The total protein amount in the bronchoalveolar lavage fluid was measured by Dye-binding assay (Bio-Rad Laboratories, Hercules, CA). Furthermore, the data were statistically evaluated by ANOVA.

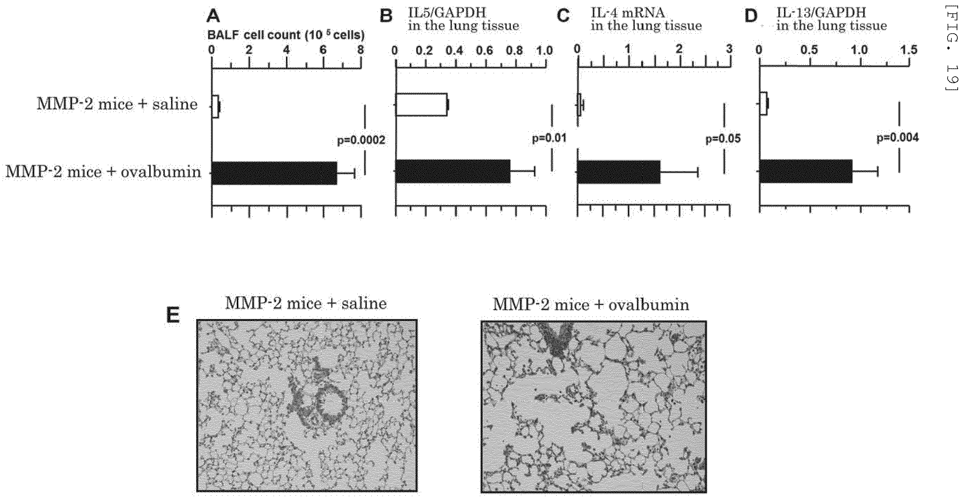

The results are shown in Fig. 19. The group sensitized with ovalbumin (MMP-2 mice + ovalbumin) had a significantly high total cell number in the bronchoalveolar lavage fluid (BALF) (A) and showed significantly high expression levels of interleukin-5 (IL5; B), interleukin-4 (IL4; C) andinterleukin-13 (IL13; D) in the lung tissue compared to the control group receiving saline (MMP-2 mice + saline) . The mice sensitized with ovalbumin also showed significantly higher degrees of bronchitis and emphysema (E) compared to the control group.

As mentioned above, the hMMP2-expressing TG mouse group had large-scale destruction of the alveolar wall after administration of ovalbumin, indicating frequent developments of COPD.

As mentioned above, based on this patent application, TG mice systemically expressing hMMP2 can be provided. Since the TG mice spontaneously develop COPD, research on COPD can be dramatically advanced. Note that cigarette smoke, the cigarette smoke extract or cigarette component, albumin and bleomycin were used as inducers for this patent, but other inducers may include environmental pollutants, proteases, etc., and a combination or several inducers can also be used. In addition, inhalation of cigarette smoke and its extract or albumin, and intraperitoneal administration of bleomycin were used as method for induction in this patent, but other methods can be used including intravenous injection, oral administration, subcutaneous administration, transtracheal administration, etc.

## Claims

1. A transgenic (TG) mouse expressing human matrix metalloproteinase 2 (hMMP2) -that includes in its structure a CMV enhancer, a chicken β-actin promoter and the whole gene region of hMMP2 and which is placed on the downstream of the promoter to make possible its systemic expression.

2. A method for producing an human matrix metalloproteinase 2 (hMMP2)-expressing transgenic (TG) mouse in which the hMMP2 expression construct is prepared by incorporating the whole gene region of the hMMP2 in the downstream of a CMV enhancer and a chicken β-actin promoter to make possible the gene expression; the hMMP2 expression construct is introduced into a fertilized egg suitable for implantation into a mouse.

3. A method for producing an animal model for developing diseases such as chronic obstructive pulmonary disease (COPD), pulmonary fibrosis, airway remodeling and pulmonary hypertension, by administration of an inducer to the hMMP2 expressing TG mouse as claimed in claim 1, wherein the inducer is cigarette smoke, a cigarette smoke extract, a cigarette component, bleomycin or albumin.

## Patentansprüche

1. Transgene (TG-) Maus, die menschliche Matrix-Metalloproteinase 2 (hMMP2) exprimiert, die in ihrer Struktur einen CMV-Enhancer, einen Huhn-β-Actinpromotor und die gesamte Genregion von hMMP2 umfasst, welche stromab des Promotors angeordnet ist, um ihre systemische Expression zu ermöglichen.

2. Verfahren zur Herstellung einer menschliche Matrix-Metalloproteinase 2 (hMMP2) exprimierenden transgenen (TG-) Maus, wobei das hMMP2-Expressions-konstrukt durch Aufnehmen der gesamten Genregion des hMMP2 in den Stromabbereich eines CMV-Enhancers und eines Huhn-β-Actinpromotors hergestellt wird, um die Genexpression zu ermöglichen; wobei das hMMP2-Expressionskonstrukt in ein befruchtetes Ei eingeführt wird, das für Implantation in eine Maus geeignet ist.

3. Verfahren zur Herstellung eines Tiermodells zur Entwicklung von Erkrankungen wie beispielsweise chronisch obstruktiver Lungenerkrankung (COPD), Lungenfibrose, Atemwegsremodellierung und Lungenhochdruck durch Verabreichen eines Induktors an die hMMP2 exprimierende TG-Maus nach Anspruch 1, wobei der Induktor Zigarettenrauch, ein Zigarettenrauchextrakt, eine Zigarettenkomponente, Bleomycin oder Albumin ist.

## Revendications

1. Souris transgénique (TG) exprimant la métalloprotéinase matricielle 2 humaine (hMMP2) - qui inclut dans sa structure un activateur de CMV, un promoteur de β-actine de poulet et la région génique entière de la hMMP2, et qui est placée en aval du promoteur pour rendre possible son expression systémique.

2. Procédé pour produire une souris transgénique (TG) exprimant la métalloprotéinase matricielle 2 humaine (hMMP2), dans lequel la construction d'expression de hMMP2 est préparée en incorporant la région génique entière de la hMMP2 en aval d'un activateur de CMV et d'un promoteur de β-actine de poulet pour rendre possible l'expression génique ; la construction d'expression de hMMP2 est introduite dans un ovule fécondé adapté pour une implantation dans une souris.

3. Procédé de production d'un modèle animal pour développer des maladies telles que la maladie pulmonaire obstructive chronique (COPD), la fibrose pulmonaire, le remodelage des voies aériennes et l'hypertension pulmonaire, par administration d'un inducteur à la souris TG exprimant la hMMP2 selon la revendication 1,
dans lequel l'inducteur est une fumée de cigarette, un extrait de fumée de cigarette, un composant de cigarette, la bléomycine ou l'albumine.
